# EUROPEAN PATENT APPLICATION

(11) **EP 1 553 188 A1**
(43) Date of publication of application: **13.07.2005**
(21) Application number: 03758115.4
(22) Date of filing: 10.10.2003
(51) Int. Cl.: C12Q 1/34

(54) **USE OF 4-GALACTOSYL-XYLOSE IN HUMANS FOR THE IN VIVO EVALUATION OF INTESTINAL LACTASE AS A NON-INVASIVE DIAGNOSTIC TEST OF THE DEFICIENCY OF SAID ENZYME**

(30) Priority: 16.10.2002 ES 200202372
(71) Applicant: CONSEJO SUPERIOR DE INVESTIGACIONES CIENTIFICAS, 28006 Madrid (ES); Universidad Autonoma de Madrid, 28049 Madrid (ES)
(72) Inventor: ARAGON REYES, Juan José, Univ. Autonoma de Madrid, Arzobispo Morcillo, 28029 Madrid (ES); FERNANDEZ MAYORALAS ALVAREZ, Alfonso, 28006 Madrid (ES)
(74) Representative: Ungria Lopez, Javier
(86) International application number: PCT/ES2003/000517
(87) International publication number: WO 2004/035814

(57) **Abstract**

The invention relates to the use in humans of a method which is based on the use of 4-galactosyl-xylose and which involves an alternative technique to evaluate xylose in urine. The method consists of measuring the concentration of xylose in the blood following the oral administration of 4-galactosyl-xylose to the patient, the use of said method being even more suitable in the case of newborns. The invention also relates to the technology used for the *in vivo* evaluation of intestinal lactase activity, by way of a non-invasive test for the diagnosis of a deficiency of said enzyme, which consists of directly evaluating the overall activity of the enzyme in the individual and not in assessing the consequences of said deficiency. The inventive method does not require specialized equipment, does not cause any obvious discomfort to patients with a lactase deficiency and is extremely reliable, thereby eliminating the inconveniences of currently used diagnostic tests.

## Description

### TECHNICAL FIELD

The invention fits in the pharmaceutical sector and is applicable in the medical sector in the *in vivo* evaluation of intestinal lactase activity in humans as a non-invasive diagnostic test of the deficiency of this enzyme, as well as in any other normal or pathological circumstance when the levels of this enzyme in vivo need to be evaluated.

### PRIOR ART

Intestinal lactase is found in the mucous membrane of the small intestine with its active center exposed towards the lumen of the intestine and it is the enzyme responsible for the hydrolysis of lactose, the sugar present in milk. This sugar is not absorbed as such at the intestinal level and therefore, prior hydrolysis into its components galactose and glucose, which are the ones finally absorbed, is essential. The deficiency of intestinal lactase leads to lactose malabsorption and intolerance. Due to the deficiency of this enzyme, the lactose not hydrolyzed in the small intestine causes a delay in the stomach emptying rate and an increase of the intestinal transit, an increase of osmotic pressure and retention of fluid in the intestinal lumen, giving rise to bacterial fermentation thereof in the colon, the production of gases such as hydrogen, methane and carbon dioxide and the formation of short-chain fatty acids. As a result of these facts, the digestion process is decreased and the absorption of monosaccharides is reduced. This causes pain, stomach cramps, flatulence, audible intestinal noise and diarrhea. In adults this clinical picture is known as hypolactasia or lactose intolerance, which is the most frequent genetic disorder, affecting around half of mankind. In the case of newborns, the congenital deficiency of the enzyme prevents correct use of lactose, giving rise to severe disorders, such as intense diarrhea and dehydration, derived both from the reduction of the energy supply as well as from the accumulation of non-hydrolyzed disaccharide in the intestine, which requires early detection and a diet change to milk without lactose. Besides, lactase deficiency has a secondary form in an important number of intestinal pathologies that involve a deterioration of the intestinal mucous membrane in different degrees, such as celiac disease, chronic intestinal inflammatory disease (Crohn's disease and ulcerous colitis), spastic colon syndrome, intestinal resections, cystic fibrosis, premature newborns, administration of chemotherapy, or as an additional disorder in old people, among others. Evaluation of lactase activity is, thus, of particular interest in gastroenterology, pediatrics, and, in general, in pathological processes wherein it is necessary to evaluate the functional integrity of the intestinal mucous membrane or to make a differential diagnosis of the deficiency of this enzyme. Up to now, diagnosis of the deficiency of intestinal lactase has been based on:
- Direct evaluation of the lactase enzyme of a sample of the patient's mucous membrane of the small intestine obtained by means of a biopsy by endoscopy (1).
- Evaluation of the metabolic consequences derived from the deficiency of the enzyme after a test of an oral overdose of lactose (1-2 g per kg of body weight), such as the apperance of symptoms of deficiency in the individual (abdominal pain, flatulence, diarrhea, etc.) and evaluation of glucose in plasma (2), of galactose in plasma (2) and urine (3) and carbohydrates in feces (4). Evaluation of the gases exhaled after an overdose of lactose, gases resulting from fermentation in the colon of non-metabolized lactose, such as the evaluation of hydrogen exhaled in the breath (5), of ¹⁴CO₂ (6) or ¹³CO₂ (7) after the overdose of lactose containing ¹⁴-C-lactose or ¹³C-lactose, respectively, or else of the ratio ¹³CO₂/H₂ (8). Evaluation in serum of the ratio ¹³C/²H-glucose (9) after the overdose with lactose containing ¹³C-lactose and ²H-glucose.

Other methods for evaluating intestinal lactase are based on the use of certain disaccharides that are structural analogues of lactose and that are therefore, capable of functioning as substrates of this enzyme. Hence, after the intake thereof, they are converted by the action of intestinal lactase into certain monosaccharides that are absorbed by the intestinal mucous membrane and some of them can be evaluated by the excretion of one or some of them in urine. Hence, the methods described in Spanish patents ES-P-478590 and ES-P-482073 are based on *in vivo* evaluation by means of oral administration of 3-O-methyl lactose and the evaluation of 3-O-methyl-D glucose in urine.

On the other hand, Spanish patent ES-P-9001680 describes the preparation of 4-O-β-galactopyranosyl-D-xylose disaccharide of the formula 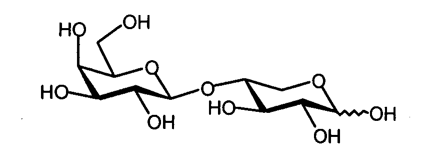 for *in vivo* evaluation of intestinal lactase activity. Said disaccharide is administered orally, acts as an intestinal lactase substrate and therefore, it hydrolyzes in the intestinal tract into xylose and galactose, both being absorbed and the xylose being excreted in urine where it can be directly titrated by a simple colorimetric method. The amounts of xylose excreted are correlated with the levels of intestinal lactase.

Spanish patent ES-P-9001680 also describes a method for preparing 4-O-β-galactopyranosyl-D-xylose that comprises synthesis from benzyl β-D-xylopyranoside which implies reactions of selective protection, glycosylation and deprotection.

Spanish patent ES-P-9502185 describes enzymatic processes for preparing mixtures of galactopyranosyl-xylose disaccharides that contain 4-O-β-galactopyranosyl-D-xylose and regioisomers thereof 2-O-β-galactopyranosyl-D-xylose and 3-O-β-galactopyranosyl-D-xylose. Spanish patent ES-P-9502185 also describes the use of a mixture of the three cited regioisomers in *in vivo* evaluation of intestinal lactase activity in unweaned rats, demonstrating that the excretion of xylose in urine after oral administration of this mixture to animals during their growth is proportional to the intestinal lactase activity evaluated *postmortem* in the intestinal mucous membrane of the same animals.

Spanish patent ES-P-20010419 describes an enzymatic process to obtain 4-O-β-galactopyranosyl-D-xylose that implies an enzymatic reaction between D-xylose and a β-D-xylopyranoside substrate and a subsequent step of isolation and purification of 4-O-β-galactopyranosyl-D-xylose.

An object of this patent is to describe the use of 4-O-β-galactopyranosyl-D-xylose prepared according to the process described in Spanish patent ES-P-20010419 for *in vivo* evaluation of intestinal lactase activity in humans as a non-invasive diagnostic test of the deficiency of this enzyme. An alternative method to the one described in prior patents based on the evaluation of xylose in blood is also used in the present patent, which involves substantial advantages for applying the diagnostic test.

### References

1.Dahlqvist, A. (1964). Anal. Biochem. 7, 18-25.
2.Newcomer, A.D., McGill, D.B., Thomas, P.J. & Hofmann, A.F. (1975) N. Engl. J. Med. 293, 1232-1236.
3.Goda, T., Bustamante, S. Thornburg, W. & Koldovsky, O. (1984) Biochem. J. 221, 261-263.
4.Kerry, K.R. & Anderson, C.M. (1964) Lancet *1*, 981-984.
5.Metz, G., Jenkins, D.J., Peters, T.J., Newman, A. & Blendis, L.M. (1975) Lancet *1*, 1155-1157.
6.Sasaki, Y., Lio, M., Kameda, H., Ueda, H. & Aoyagi, T. (1970) J. Lab. Clin. Med. 76, 824-835.
7.Koetse, H.A., Stellaard, F., Bijleveld, C.M., Elzinga, H. Boverhof, R., Van der Meer, R., Vonk, R.J. & Sauer, P.J. (1999) Scand. J. Gastroenterol. 34, 35-40.
8.Koetse, H.A., Vonk, R.J., Pasterkamp, S., Pal, J., de Brujin, S. & Stellard, F. (2000) Scand. J. Gastroenterol. 35, 607-611.
9.Vonk, R.J., Stellaard, F. Priebe, M.G., Koetse, H.A., Hagedoorn, R.E., de Brujin, S., Elzinga, H., Lenoir-Wijnkoop, I. & Antoine, J.M. (2001) Eur. J. Clin. Invest. 31, 226-233.

### DESCRIPTION OF THE INVENTION

The methods used nowadays to diagnose the deficiency of intestinal lactase, described in the preceding section, involve a series of disadvantages that in general imply scarce reliability and significant discomfort in patients, aside from the need of specialized equipment as described hereinafter. Direct evaluation of lactase activity in a patient's sample of mucous membrane of the small intestine obtained by biopsy by means of endoscopy, aside from being a bloody and invasive test that requires X-ray control, only indicates the enzyme activity in one specific part or area of the intestine. Besides, it does not generally coincide with the area of the largest presence of this enzyme (the middle jejunum), due to difficult access by means of endoscopy and it is also variable from some samples to others. Hence, information regarding the total enzyme activity in the individual is not provided. The rest of the methods are non-invasive tests that imply an oral overdose of lactose (from 25 to 50 g) and subsequent evaluation of the metabolic consequences of the lactase deficiency, such as measuring in the breath the gases coming from the metabolization of the lactose not hydrolyzed by the bacteria found in the large intestine. These tests are indirect, given that they do not permit direct *in vivo* measurement of the activity of the enzyme and they depend on the capacity of endogenous production of gases, such as H₂ or CO₂ by the individual. Besides, this production capacity may be variable from some individuals to others since it is affected by different factors such as the habit of smoking, diet, use of laxatives or oral antibiotic therapy, therefore, a significant proportion of positive false results and negative false results are produced and hence, the reliability thereof is scarce. On the other hand, the tests with a lactose overdose require the intake of amounts of sugar much higher than the ones present in a physiologically balanced diet, which causes considerable gastrointestinal problems in individuals with a deficiency, such as abdominal pain, flatulence and diarrhea. In unweaned babys with a presumed deficiency, the overdose of lactose may be dangerous due to the cause of significant diarrhea and potential dehydration, which obviously requires another type of test. Use of tests of the overdose of lactose, in the different types thereof, require specialized equipment, such as gas chromatographs or electrochemical detectors to measure the gases in the breath, which although they are usually found in hospitals, they are not found in health centers in general. Use of sparkling counters or much more specialized and limited equipment such as gas chromatography or mass spectrometry to evaluate ¹⁴CO₂, ¹³CO₂ and ¹³C/²H-glucose is also required, when the overdose implies the use of 1-¹⁴C-glucose or ¹³C-lactose/²H-glucose. Oral administration of 3-O-methyl lactose and the subsequent evaluation of 3-O-methyl-D-glucose excreted in urine, not used up to now in clinical practice, implies on the one hand, absorption into the blood of a non-physiological compound, such as 3-O-methyl-D-glucose, and requires gas chromatography or high pressure liquid chromatography systems for the evaluation thereof.

### OBJECT OF THE INVENTION

To overcome the inconveniences of the currently used diagnostic tests described in the preceding paragraph by providing a technology for *in vivo* evaluation of intestinal lactase activity, as a non-invasive diagnostic test of this enzyme, that is based on direct evaluation of the overall activity in the enzyme in the individual as a whole. It is not an evaluation of the consequences of the deficiency thereof. No specialized equipment is required and no obvious discomfort is caused to patients with a lactase deficiency and the technology is highly reliable, among other advantages that will be described in the following paragraph.

To use the method based on the use of 4-galactosyl-xylose in humans and integrating in this method an alternative technique for evaluating xylose in urine, consisting of evaluating the concentration of xylose in blood after administering 4-galactosyl-xylose to the patient. Extracting a minimum volume of blood per sample (2-3 drops) suffices and this proves to be more convenient when the method is used in children and newborns since the method produces less discomfort to these individuals and is more reliable.

### BRIEF DESCRIPTION OF THE INVENTION

The methodology object of the invention is based on the use of 4-galactosyl-xylose (4-O-β-galactopyranosyl-D-xylose). This compound acts as a structural analogue of lactose, the physiological substrate of lactase, in such a way that after oral administration thereof, it is hydrolyzed by the enzyme of the intestinal mucous membrane. The products of the reaction pass into the blood and one of them, xylose, appears in urine, where it may be titrated by a simple colorimetric evaluation. Alternatively, the present invention describes the evaluation of xylose in blood, for which purpose a few drops of blood suffice, given the high sensitivity of the xylose evaluation method. This alternative implies a substantial advantage especially in the case of unweaned babys and children in general, given that the taking of urine samples during several hours is avoided and the alternative produces much less discomfort and provides higher reliability. This invention shows that the intake of 4-galactosyl-xylose by humans with lactose tolerance is followed by the presence of xylose to be present in urine and in blood, which is the expression of intestinal lactase activity in these individuals. Likewise it shows that the excretion of xylose in urine, as well as the concentration of xylose in blood, is reduced after administering 4-galactosyl-xylose to humans with an intestinal lactase deficiency (lactose intolerance). Furthermore, for this first time it is evidenced that the use of 4-galactosyl-xylose and the evaluation of xylose in urine makes it possible' to reliably and simply distinguish between individuals having a lactase deficiency as opposed to individuals with an allergy to some component of milk. In comparison with the methods used nowadays to diagnose intestinal lactase deficiency, the method that is described in this invention offers the following advantages:

High reliability, given that this methodology is based on the direct evaluation of the enzyme activity by means of evaluating xylose in urine or plasma, not on evaluating the metabolic consequences derived from the deficiency thereof, such as the production of H₂ or CO₂ resulting from the fermentation of the non-hydrolyzed disaccharide in the colon. Consequently, all those circumstances described in the first paragraph of this section, that affect endogenous production of gases and that therefore significantly reduce the reliability of the tests measuring gases in the breath after an overdose of lactose, frequently producing false positive and false negative results, do not have any influence on the results that are obtained with the invention described herein.

The information provided is directly indicative of the total in vivo lactase activity in the individual.

High sensitivity: the minimum limit of detection of xylose when it is titrated by colorimetric analysis based on the reaction of fluoroglucinol is from 0.1 to 0.05 µg.

Simplicity of the required equipment, normally available in any health center with the minimum facilities of a clinical biochemical laboratory, since the *in vivo* evaluation of the enzyme is based only on the titration of xylose, which can be done by a simple colorimetric test.

Absence of adverse gastrointestinal discomfort or disorders in patients with lactase deficiency when the test is carried out, due to the fact that the oral dose of 4-galactosyl-xylose disaccharide that is administered is very small, which in adults is 0.25 g when the amount of xylose in urine is evaluated and 3.0 g when the amount of xylose in blood is evaluated. This makes it possible to foresee that the doses administered to children are even smaller. Reduction of the dose of the disaccharide is a consequence of the high sensitivity of the xylose evaluation. It is also noteworthy that hydrolysis of 4-galactosyl-xylose by intestinal lactase gives rise to the production of two physiological products, galactose and xylose, that are finally absorbed by the intestinal mucous membrane.

No active participation on the part of the individual is required and this proves to be of particular interest in unweaned babys.

No compounds marked with radioactive isotopes need to be administered.

### DESCRIPTION OF THE FIGURES

**Figure 1:** Excretion of xylose in urine after oral administration of 4 mg of 4-galactosyl-xylose (black circle) and intestinal lactase activity (white circle) in unweaned rats during growth.
**Figure 2**: Levels of xylose in plasma after oral administration of 8 mg of 4-galactosyl-xylose (black circle) and intestinal lactase activity (white circle) in unweaned rats during growth.
**Figure 3**: Excretion of xylose in urine by an adult human volunteer with a tolerance to the intake of milk (individual 1, male) after oral administration of 1 g of 4-galactosyl-xylose.
**Figure 4**: Excretion of xylose in urine by human volunteers over time after oral administration of different doses of 4-galactosyl-xylose to two adult human volunteers with a tolerance to the intake of milk (individual 1, male; individual 2, female).
**Figure 5**: Excretion of xylose in urine by human volunteers over time after oral administration of 250 mg of 4-galactosyl-xylose to three males [individuals 1 (black circle, white circle), 7 (black triangle, white triangle) and 11 (black square, white square)] and five females [individuals 2 (black circle, white circle), 3 (black square, white square), 6 (black diamond, white diamond), 8 (black reversed triangle, white reversed triangle) and 9 (black triangle, white triangle) with tolerance to the intake of milk.
**Figure 6**: Excretion of xylose in urine over time after oral administration of 0.25 g of 4-galactosyl-xylose to three human volunteers [individuals 12 (male), 5 (female) and 10 (female)] with intolerance to the intake of milk.
**Figure 7**: Evaluation of xylose in urine by gas chromatography in individual 1, with tolerance to the intake of milk and in individual 12, with intolerance to the intake of milk after the intake of 0.28 g of 4-galactosyl-xylose.
**Figure 8**: Levels of xylose in blood plasma of adult human volunteers after oral administration of 3 g of 4-galactosyl-xylose to individuals 1 (white circle) and 2 (black circle) with normal tolerance to the intake of milk and to the hypolactasic individual 12 (black square).

### DETAILED DESCRIPTION OF THE INVENTION

The methodology consists of the oral administration of an aqueous solution of 4-galactosyl-xylose to humans after at least 8 hours of fasting. The amount of xylose present in the urine excreted is evaluated as of then, after a specific period of time. The methodology can be carried out likewise by evaluating the presence of xylose in blood evaluating it in plasma, or blood serum, prepared from a blood sample extracted after a period of time, after the intake of the disaccharide. The titration of xylose can be done by means of colorimetric analysis based on the reaction with fluoroglucinol and using as a target, either the basal urine or else the basal plasma or the basal blood serum, depending on the methodology that is used. Once the intake of 4-galactosyl-xylose has taken place, it is hydrolyzed by the intestinal lactase, in such a way that the products of this enzymatic reaction, galactose and xylose, are absorbed into the blood through the intestinal mucous membrane. Later xylose is excreted in urine and hence the amount of xylose that appears in the blood and in the urine after the intake of the disaccharide, is a direct expression of the *in vivo* activity of the enzyme.

The 4-galactosyl-xylose used to carry out this invention was prepared according to the method described in Spanish patent ES-P-20010419.

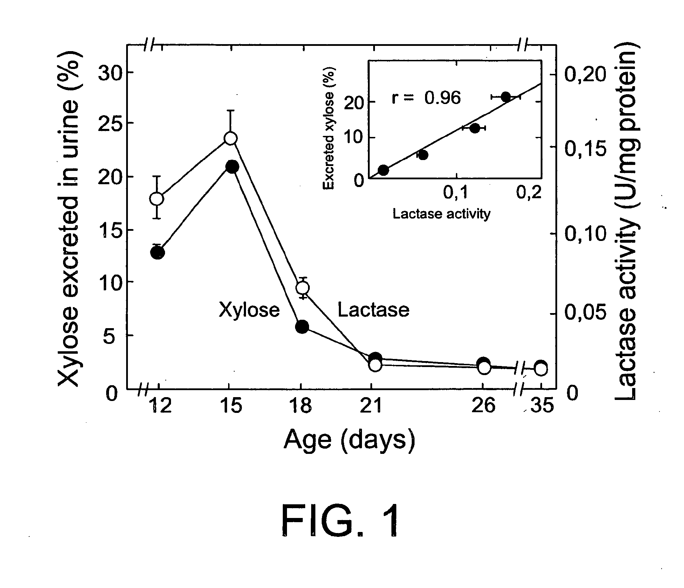

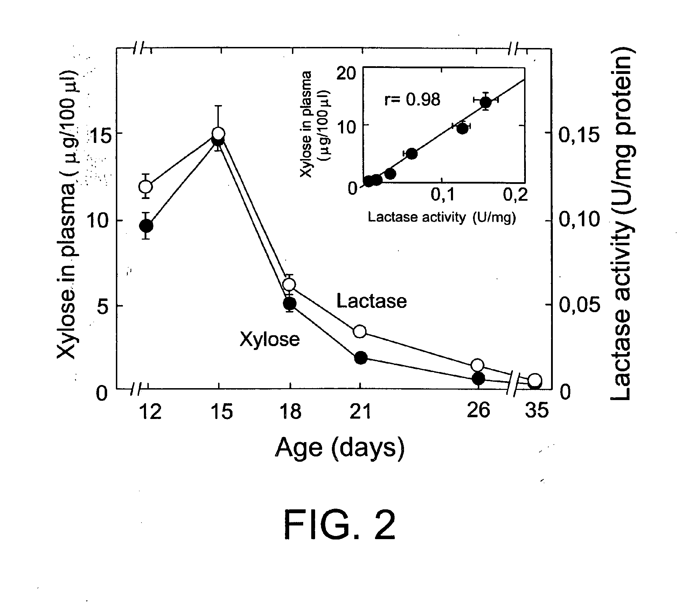

The excretion of xylose in urine, as well as the presence of xylose in blood, after oral administration of 4-galactosyl-xylose closely correlate with intestinal lactase activity, as it has been examined in unweaned rats, are represented in Figs. 1 and 2 and described in Examples 1 and 2.

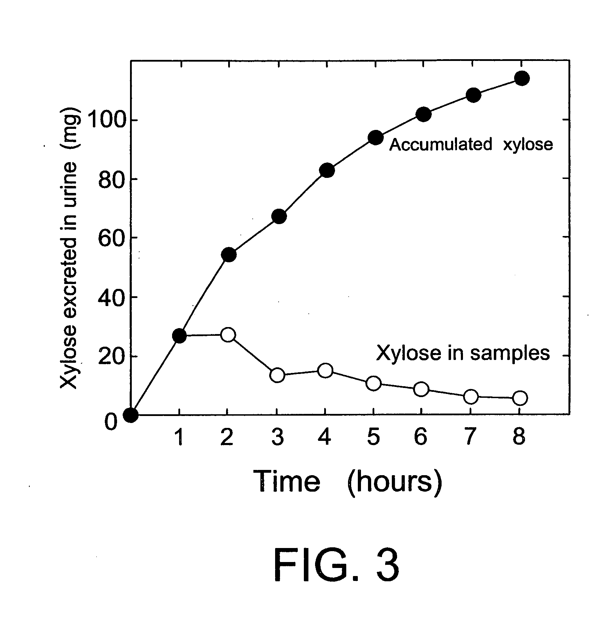

The intake of 4-galactosyl-xylose by adult human volunteers with a normal digestion capacity of lactose or tolerance of lactose, in other words, who have normal levels of intestinal lactase activity, judged on the basis that no type of adverse gastrointestinal discomfort or disorder was stated, after there was an intake of milk or any other milk product, gave rise to a gradual excretion of xylose in urine over a period of 8 hours after the disaccharide had been administered, as a result of the hydrolysis thereof by intestinal lactase. This shows that 4-galactosyl-xylose is an *in vivo* substrate of human intestinal lactase, as represented in Figs. 3, 4 and 5 and as described in Examples 3 to 11.

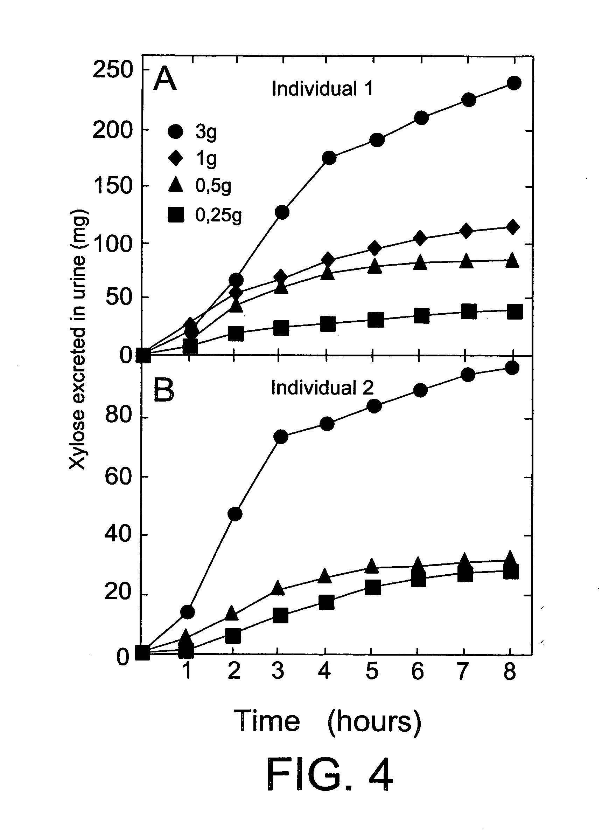

Excretion of xylose in urine by adult human volunteers with lactose tolerance increased with the amount of the intake of 4-galactosyl-xylose, when it was used at doses of 0.25 g, 0.5 g, 1 g and 3 g. The intake of 4-galactosyl-xylose did not cause any gastrointestinal discomfort or disorder in the individuals studied. This shows that the excretion of xylose in individuals is dependent on the dose of 4-galactosyl-xylose that is administered. A dose of 0.25 g of 4-galactosyl-xylose suffices to reliably detect xylose in urine after the intake of the cited compound, as represented in Fig. 4 and described in Examples 4 and 5.

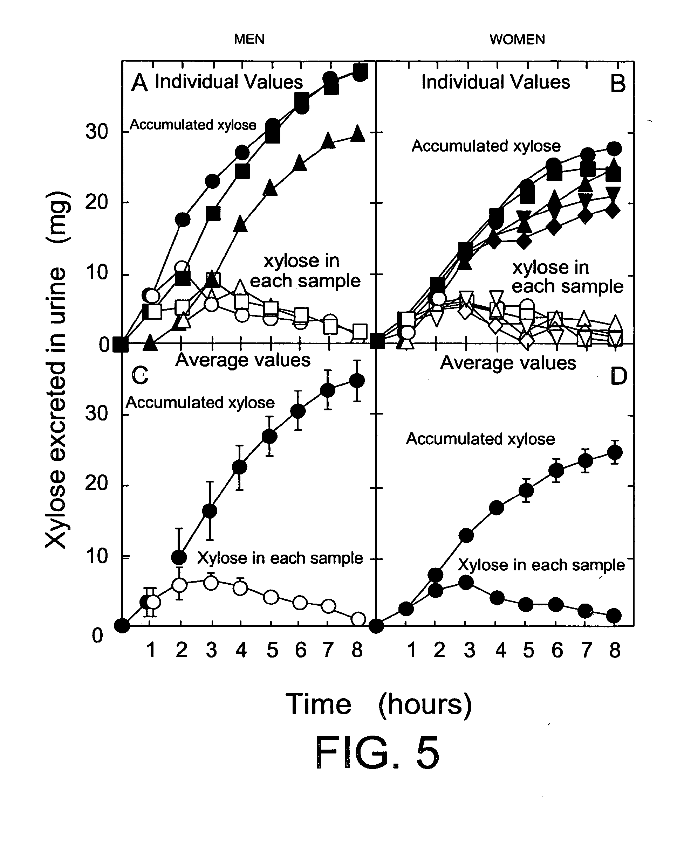

The proportion of xylose excreted in urine by adult human volunteers with lactose tolerance was within the same range when the same amount of 4-galactosyl-xylose was administered to individuals of the same sex. This shows that the results obtained with this methodology are reliably reproduced in some normal individuals to others, as represented in Fig. 5 and described in Examples 4 to 11.

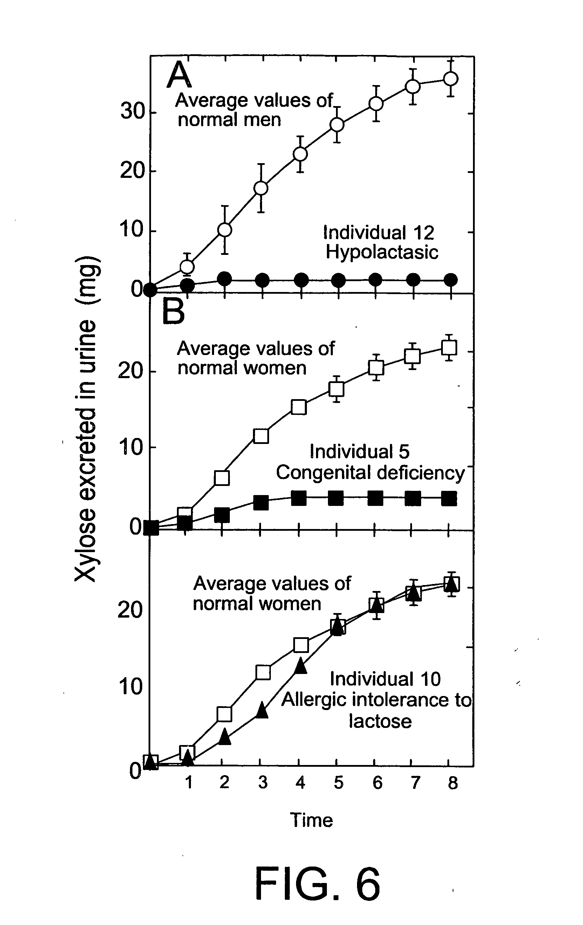

Administration of 0.25 g of galactosyl-xylose to adult human volunteers with lactose intolerance, carriers of a intestinal lactase deficiency, gives rise to a significant reduction of xylose excreted in urine with respect to the average values observed in volunteers with lactose tolerance, in terms of the degree of enzyme deficiency. This was observed in two individuals studied who had lactose intolerance, one of them previously diagnosed as intensely hypolactasic and the other who stated that he had lactose intolerance since birth, as represented in Figs. 6A and 6B, respectively and as described in Examples 12 and 13, respectively. The intake of 4-galactosyl-xylose by these two individuals with lactose intolerance did not cause them any type of gastrointestinal discomfort or disorders. This shows that the methodology that is presented permits *in vivo* evaluation of intestinal lactase activity and it is a valid test for non-invasive diagnosis of the deficiency of this enzyme in humans. It does not cause obvious discomfort in individuals having this deficiency and a dose of merely 0.25 g of 4-galactosyl-xylose suffices to reliably detect intense deficiencies of the enzyme.

Oral administration of 4-galactosyl-xylose and the subsequent evaluation of the resulting xylose in urine makes it possible to distinguish between patients with lactose intolerance who are carriers of an intestinal lactase deficiency and patients with an intolerance to the intake of milk who are so because they have symptoms of allergy to the components of milk but who are not carriers of the deficiency of this enzyme, given that in the latter, the excretion of xylose in urine is within normal limits. This is shown in Fig. 6C and is described in Example 14. This Figure 6C shows that the excretion of xylose in urine after an intake of 0.25 g of 4-galactosyl-xylose by an adult volunteer with lactose intolerance, who stated having suffered from allergic skin reactions after the intake of milk or milk products since childhood, was not significantly different from the behavior observed by the average of female volunteers with lactose tolerance studied. The intake of 4-galactosyl-xylose by this adult female volunteer with allergic lactose intolerance did not cause any allergic reaction or any type of gastrointestinal disorder.

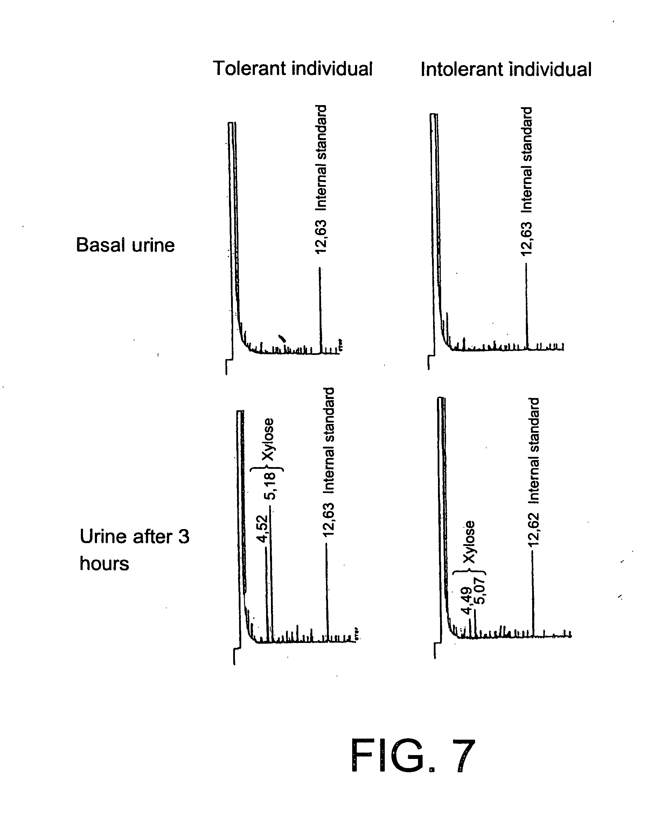

Excretion of xylose in urine after administering 0.25 g of 4-galactosyl-xylose to individuals with lactose tolerance and the reduction thereof in individuals with hypolactasic lactose intolerance is additionally shown by a xylose evaluation technique different from the colorimetric reaction with fluoroglucinol, such as gas chromatography. This is shown in Fig. 7 and described in Example 15, wherein it is likewise shown that the excretion of xylose in urine is significantly reduced when 4-galactosyl-xylose is administered to a hypolactasic individual with lactose intolerance in comparison to an individual who has lactose tolerance.

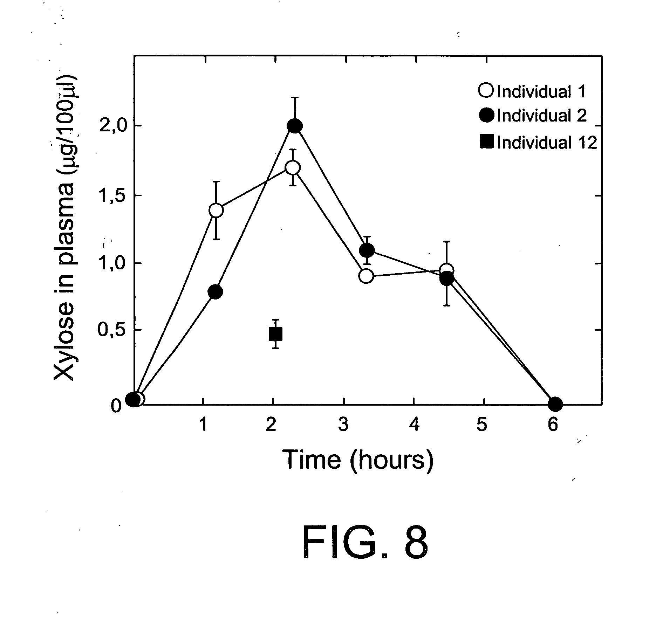

Oral administration of 3 g of 4-galactosyl-xylose to adult human volunteers with lactose tolerance gave rise to the gradual presence of xylose in blood plasma titrated colorimetrically with fluoroglucinol, that reached a maximum about 2 hours after the intake of the disaccharide. Oral administration of the same amount of 4-galactosyl-xylose to an adult human volunteer with lactose intolerance diagnosed as hypolactasic produced a significant reduction in the amount of xylose evaluated in blood plasma of this individual two hours after the intake of the disaccharide. This test did not give rise to any gastrointestinal discomfort or disorder in this individual with lactose intolerance. This is shown in Fig. 8, described in Example 16 and it shows that the evaluation of xylose in blood after oral administration of 4-galactosyl-xylose allows *in vivo* evaluation of intestinal lactase activity and it is a valid test for non-invasive diagnosis of the deficiency of this enzyme in humans.

Taking into account the pattern that the presence of xylose in blood and urine follows over time, after oral administration of 4-galactosyl-xylose to human volunteers with lactose tolerance or lactose intolerance and the high sensitivity of the method of colorimetric titration of xylose with fluoroglucinol used, the samples of body fluids to be used with this methodology in adult individuals can be basal urine before the intake of 4-galactosyl-xylose and total urine 3 or 4 hours after the intake of this compound, or else a sample of basal blood before the intake of 4-galactosyl-xylose and another sample of blood extracted 2 hours after the administration of this compound. A volume of 0.2 ml of blood (2-3 drops) suffices in each sample.

This test is likewise applicable to children and unweaned babys, evaluating xylose in urine as well as in blood. The dose of 4-galactosyl-xylose to be orally administered can be reduced in half and the use of blood samples of 0.2 ml (2-3 drops), can suffice as a first approximation.

The evaluation of xylose in blood represents a substantial advantage as it can be reliably carried out with 50µl of serum or plasma, for which 2-3 drops of blood per sample suffice. This is especially of interest in unweaned babys and children in general, wherein tests that require the total urine of several hours to be collected make it difficult to be sure of the volume of urine collected and the possible production of skin reactions in the perineal area. This evaluation method produces less discomfort and is more reliable.

### EMBODIMENTS OF THE INVENTION

**Example 1:** Correlation between the excretion of xylose in urine and the presence of xylose in blood after oral administration of 4-galactosyl-xylose with intestinal lactase activity. This has been shown in unweaned rats comparing during the growth of these animals, the excretion of xylose in urine after orally administering to them 4-galactosyl-xylose, with lactase activity evaluated *postmortem* in the intestinal mucous membrane, which is known to physiologically reduce during growth. For this purpose, a group comprised of 20 12-day old unweaned rats belonging to two litters fasted for 6 hours by being separated from the mother. After this period of time passed, basal urine was collected from each animal by transabdominal vesical pressure and each one was immediately administered 4 mg of 4-galactosyl-xylose diluted in 0.3 ml of distilled water by using an intraesophageal probe. From this moment urine was collected during the following 6 hours and the xylose excreted therein was titrated by colorimetric analysis based on the reaction with fluoroglucinol and using basal urine as the target. Immediately after the urine has been collected, four of the animals were sacrificed and the lactase activity in the intestinal mucous membrane was directly evaluated. For this purpose a piece of small intestine was resected, washed and the mucous membrane was collected by scraping with glass and the scraped mucous membrane was homogenized. The lactase activity in the homogenized product was measured spectrophotometrically. The remaining animals were returned to the mother and the experiment was repeated under similar conditions at the age of 15, 18, 21, 26 and 35 days when the initial animal population was sacrificed. The results of this experiment are compiled in Fig. 1. Therein one can see: 1) That the presence of xylose in urine coming from the hydrolysis of the 4-galactosyl-xylose administered is detected by the action of intestinal lactase; 2) That the rate of excretion of xylose in urine caused by oral administration of 4-galactosyl-xylose during the growth of the animals, runs parallel to the known physiological modification of intestinal lactase activity that reduces during growth. The excretion of xylose in urine is closely correlated to intestinal lactase activity as shown by the linear correlation coefficient (r) of 0.96 obtained between both parameters, as represented in the insert of Fig. 1. Prior to this experiment it was found that the excretion of xylose in urine increases with the increase of the amount of 4-galactosyl-xylose administered orally to animals despite their age, varying the doses of this disaccharide from 2 to 32 mg diluted in 0.3 ml of water. This experiment shows that the methodology that is presented is useful for *in vivo* evaluation of intestinal lactase activity. The process can be used for non-invasive evaluation of this activity in a bloodless manner for diagnostic purposes.
**Example 2**. Correlation between the excretion of xylose in urine and the presence of xylose in blood after oral administration of 4-galactosyl-xylose with intestinal lactase activity. This has been shown in unweaned rats by comparing during the growth of these animals the amount of xylose in plasma analyzed after orally administering to them 4-galactosyl-xylose with specific lactase activity evaluated *postmortem* in the intestinal mucous membrane. For this purpose, a group comprised of 60 12-day old unweaned rats belonging to two litters fasted for 6 hours by being separated from the mother. After this period of time passed, basal blood was extracted from each animal by means of a heart puncture and each rat was immediately administered 8 mg of 4-galactosyl-xylose diluted in 0.3 ml of distilled water by using an intraesophageal probe. 3 hours after the disaccharide was administered another blood sample was extracted. The blood plasma or supernatant fraction was obtained by centrifuging this sample and the xylose therein was titrated by colorimetric analysis with fluoroglucinol and using the plasma of the basal blood as the target. Immediately after the blood was extracted, four of the animals were sacrificed and the lactase activity in the intestinal mucous membrane was directly evaluated in the same manner as the one described in Example 1. The remaining animals were returned to the mother and the experiment was repeated under similar conditions at the age of 15, 18, 21, 26 and 35 days when the initial animal population was sacrificed. The results of this experiment are compiled in Fig. 2. Therein one can see: 1) That the presence of xylose in blood plasma coming from the hydrolysis of the 4-galactosyl-xylose administered is detected by the action of intestinal lactase; 2) That the rate at which xylose becomes present in blood plasma caused by oral administration of 4-galactosyl-xylose during the growth of the animals, likewise runs parallel to the physiological reduction of intestinal lactase activity during growth, the presence of xylose in blood plasma being very closely correlated to the intestinal lactase activity as shown by the linear correlation coefficient (r) of 0.98 obtained between both parameters, as represented in the insert of Fig. 2. Prior to this experiment it was found that the presence of xylose in blood plasma reaches its highest level around 3 hours after the oral administration of 4-galactosyl-xylose, that the amount of xylose in plasma increases with the increase of the amount of 4-galactosyl-xylose that is administered orally to animals irrespective of the age of the animals when the dose of this disaccharide varied between 4 and 16 mg diluted in 0.3 ml of water and that the amounts of xylose in blood serum obtained after allowing the blood samples to settle for 20 minutes at room temperature and then centrifuging them were similar to the ones evaluated in plasma. This experiment shows that the evaluation of xylose in plasma or blood serum after oral administration of 4-galactosyl-xylose is likewise useful for *in vivo* evaluation of intestinal lactase activity. The process may be used for non-invasive evaluation of this activity for diagnostic purposes.

A series of embodiments of the invention carried out on different adult human volunteers, all of whom were informed in writing of the study to be carried out and they gave their written consent to voluntarily participate therein, is described hereinafter.

**Example 3**: 4-galactosyl-xylose is an *in vivo* substrate of human intestinal lactase. This has been shown in the first place by orally administering 1 g of 4-galactosyl-xylose dissolved in 50 ml of water to an adult male volunteer (individual 1) who had fasted for 8 hours and with a normal tolerance to milk and milk products, in other words, with lactose tolerance. A basal urine sample was taken from this individual immediately before the intake of 4-galactosyl-xylose and urine was again collected after the intake thereof every hour for 8 hours, each sample showing the total urine excreted in each period of time. Xylose was titrated in all the samples by means of colorimetric analysis based on the reaction with fluoroglucinol and using basal urine as the target. The intake of 4-galactosyl-xylose did not cause any gastrointestinal discomfort or disorders in this individual. The results of this experiment appear compiled in Fig. 3, where the amount of xylose evaluated in each sample is represented (white circles) and the amount of xylose that is being accumulated in urine over time is represented (black circles), calculated by adding to the amount of xylose evaluated in each sample, the amounts evaluated in all the preceding samples. In Fig. 1 one can see: 1) That xylose is excreted in the urine of this individual after oral administration of 4-galactosyl-xylose; 2) That the excretion of xylose in urine was maximum after 2 hours and it was progressively accumulated in the urine after administration of this compound. This experiment shows that 4-galactosyl-xylose is an *in vivo* substrate of human intestinal lactase and that the evaluation of xylose in urine by oral administration of 4-galactosyl-xylose is useful for *in vivo* evaluation of intestinal lactase activity in humans, the process being useful for non-invasive evaluation of this activity for diagnostic purposes.
**Examples 4 and 5.** Excretion of xylose in urine by adult human volunteers with lactose tolerance varies according to the amount of the 4-galactosyl-xylose intake. This has been shown by administering 0.25 g, 0.5 g and 3 g of 4-galactosyl-xylose to an adult male volunteer with lactose tolerance (individual 1, Example 4) and to an adult female volunteer with lactose tolerance (individual 2, Example 5) under the same conditions as those described in Example 1 and likewise titrating xylose colorimetrically with fluoroglucinol in the urine excreted by both volunteers over time after the intake of the disaccharide. A free period of around one week was left between each intake of 4-galactosyl-xylose by the same individual. The intake of 4-galactosyl-xylose at the doses used did not cause any gastrointestinal discomfort or any disorder in either of these two individuals. The results of these experiments are compiled in Fig. 4 panel A (for individual 1 and Example 4) and in Fig. 4 panel B (for individual 2 and Example 5), wherein the amount of xylose accumulated in urine after the intake of 4-galactosyl-xylose for each administered dose is represented. Fig. 4A includes the data obtained with the same individual after an intake of 1 g of the disaccharide coming from Fig. 3 so that a comparison can be made with the rest of the administered doses. In these Figures 4A and 4B one can see: 1) That the excretion of xylose in urine over time after the intake of 4-galactosyl-xylose increased as the amount administered to individual 1 (Fig. 4A), as well as to individual 2 (Fig. 4B) increased; That a dose of 0.25 g 4-galactosyl-xylose permitted
easy detection of the presence of xylose in urine in either of these two individuals. These experiments show: 1) That the excretion of xylose in urine by adult human volunteers with lactose tolerance increased with the amount of the xylose intake when it was administered orally at doses of 0.25 g, 05 g, 1 g and 3 g, without any of the doses used having caused any gastrointestinal discomfort or disorder derived from carrying out the experiment and 2) That a dose of 0.25 g of 4-galactosyl-xylose suffices to evaluate xylose in urine very reliably after the intake of this disaccharide.
Examples 6 to 11. The proportion of xylose excreted in urine by adult human volunteers with lactose tolerance is within the same range when the same amount of 4-galactosyl-xylose is administered to individuals of the same sex. This has been shown by administering 0.25 g of 4-galactosyl-xylose to 8 adult volunteers with lactose tolerance, 3 males (individual 1, Example 4; individual 7, Example 6; individual 11, Example 7) and 5 females (individual 2, Example 5; individual 3, Example 8; individual 6, Example 9; individual 8, Example 10; individual 9, Example 11) under the same conditions as those described in Example 1 and likewise titrating xylose colorimetrically with fluoroglucinol in urine excreted by individuals over time after the intake of the disaccharide. The intake of 4-galactosyl-xylose at the doses used did not cause any gastrointestinal discomfort or disorder to any of these individuals. The results of these experiments are compiled in Fig. 5 that comprises panels A, B, C and D, wherein the amount of xylose evaluated in each sample is represented (by white symbols) and the amount of xylose that accumulates in urine over time is represented (by black symbols) calculated by adding to the amount of xylose evaluated in each sample the amounts evaluated in all the preceding samples. Panel A represents the individual values obtained in each one of the male individuals (individuals 1, 3
and 6). Panel B represents the individual values obtained with each one of the female individuals (individuals 2,3,6,8 and 9). Panels C and D represent the average values ± the standard error of the average calculated for the values corresponding to the male and female individuals studied, respectively. Fig. 5A includes the data obtained from individual 1 after an intake of 0.25 g of 4-galactosyl-xylose coming from Fig. 4A; and Fig. 5B includes the data obtained from individual 2 after an intake of 0.25 g of 4-galactosyl-xylose coming from Fig. 4B, so that they can be compared with the rest of the individuals studied under the same conditions. In Fig. 5 one can see: 1) That the accumulation of xylose excreted in urine was within the same range, without exceeding the standard error of 37% on the average in males and of 8% in females and 2) That the maximum excretion of xylose took place between 2 and 3 hours after the intake of the disaccharide. These experiments show that the results obtained with this methodology are reliably reproduced in both groups of individuals.
**Examples 12 and 13**. Administration of 4-galactosyl-xylose to human volunteers with lactose intolerance and subsequent evaluation of xylose in urine permits the detection of intestinal lactase deficiency. This has been shown by administering 0.25 g of 4-galactosyl-xylose to two volunteers with the symptoms of lactose intolerance and titrating xylose colorimetrically with fluoroglucinol in the urine excreted by both individuals over time, after the intake of the disaccharide. One of them (individual 12, Example 12) was a 64 year old male with severe intolerance to the intake of milk since he was 8 years old, and previously diagnosed as hypolactasic by an intestinal biopsy as well as by lactose overdose tests. Hence, he had an intense intestinal lactase deficiency. The other individual (individual 5, Example 13) was a 30 year old female who stated having suffered from symptoms of lactose intolerance from birth. It was necessary at that moment to stop breast-feeding her and to give her lactose-free milk. Presumably she had a congenital intestinal lactase deficiency. The intake of 4-galactosyl-xylose at the doses used did not cause any gastrointestinal discomfort or disorders to either of these two individuals. The results of these experiments are compiled in Fig. 6 panel A (individual 12, Example 12) and panel B (individual 5, Example 13) where the amount of xylose that is being accumulated in urine over time in both individuals is represented (by black symbols), including in each case the curve that represents the average values of volunteers with lactose tolerance corresponding to his/her sex and taken from Figs. 5C and 5D, in order to facilitate the comparison. In Fig. 6 one can see: 1) That the excretion of xylose in urine by individual number 12 was much lower than the average values of excretion of xylose in volunteers with lactose tolerance, this individual excreting a total of 1.33 mg of xylose in urine 8 hours, after the intake of the disaccharide, which in contrast to the average value found in the studied male volunteers with lactase tolerance of 35.7 ± 2.0 mg after the same period of time, represented a reduction of 96%, which is in accordance with the previous diagnosis of intense intestinal lactase deficiency; 2) That the excretion of xylose in urine by individual 5 was much lower than the average values of xylose excretion in volunteers with lactose tolerance. This individual excreted a total of 4.08 mg xylose in urine after 8 hours after the intake of the disaccharide. This, in contrast to the average value found in the studied female volunteers with lactose tolerance of 23.5 ± 2.0 mg after the same period of time represented an 83% reduction, which indicates that this individual also has a congenital type intense intestinal lactase deficiency, since this individual presented the lactose intolerance from birth. These experiments show that the methodology that is presented permits *in vivo* evaluation of the intestinal lactase activity and non-invasive diagnosis of the deficiency of this enzyme in humans. No apparent discomfort is caused in individuals having a deficiency and a dose of 0.25 g of 4-galactosyl-xylose suffices to reliably detect intense deficiencies of the enzyme in adult individuals.
**Example 14:** Administration of 4-galactosyl-xylose to human volunteers with lactose intolerance and subsequent evaluation of xylose in urine permits the differentiation between patients with lactose intolerance that have an intestinal lactase deficiency and patients with intolerance to the intake of milk who are so because they have symptoms of allergy to the components of milk, but who do not have a deficiency of said enzyme. This has been shown by administering 0.25 g of 4-galactosyl-xylose and subsequent titration of xylose colorimetrically with fluoroglucinol in urine excreted over time by a volunteer (individual 13), a 25 year old female who had the symptoms of lactose intolerance since she was 12 years old and she also stated suffering from acute skin allergy after the intake of milk. The intake of 4-galactosyl-xylose at the dose used did not cause any gastrointestinal discomfort or disorder in this individual. The results of these experiments are compiled in Fig. 6 panel C, where the amount of xylose that is being accumulated in urine over time in this subject is represented (by black symbols). The curve that represents the average values of volunteers with lactose tolerance corresponding to his/her sex and taken from Fig. 5D is also included in each case in order to facilitate the comparison. In Fig. 6C, one can see that this individual had a practically normal pattern of xylose excretion, excreting after 8 hours the same total amount as the average of females with lactose tolerance. Hence, it is not a matter of a deficiency of this enzyme, but rather a case of an allergy to some component of milk in view of the clinical details stated by the patient. This experiment shows that the methodology that is presented permits differentiation between patients with lactose intolerance who have an intestinal lactase deficiency and patients with an intolerance to the intake of milk who are so because they have symptoms of allergy to the components of milk, but who do not have a real deficiency of this enzyme.
**Examples 15 and 16**. Excretion of xylose in urine after administration of 0.25 g of 4-galactose-xylose to subjects with lactose tolerance, and its reduction in hypolactasic individuals with lactose intolerance is likewise observed by the evaluation technique of xylose different from the colorimetric reaction with fluoroglucinol. This has been shown by administering 0.25 g of 4-galactosyl-xylose to a volunteer with lactose tolerance [individual 1 (also studied in Example 3), Example 15] on one hand, and on the other hand, to a volunteer with lactose intolerance having an intense intestinal lactase deficiency [individual 12 (also studied in Example 12), Example 16], and evaluating xylose by gas chromatography in the total urine collected 3 hours after the intake of the disaccharide with a chromatograph equipped with a flame ionization detector and a 12 m long glass spiral column with an inside diameter of 0.2 mm and a thickness of 0.3 µm. The presence of xylose in urine was detected in its trimethylsilyl derivative form. For this purpose the urine sample was lyophilized and dissolved in 20 µl of pyridine containing 1 mM of benzyl-β-D-xylopyranoside as an internal reference. Then another 20 µl of N-trimethylsilyl imidazole were added as a sililating agent. The mixture was treated with ultrasound for one minute and heated at 60°C for 30 minutes. Then 1-2 µl of the sample was injected into the gas chromatograph. The temperature of the injector and the detector used was 250°C and the temperature gradient in the column was the following: 2 minutes at 180°C and a 5°C increase per minute until 250°C was reached. This temperature was maintained for 15 minutes. The concentration of xylose in each sample was calculated from the areas of the peaks corresponding to the chromatograms. The results of these experiments are compiled in Fig. 7, wherein the chromatograms corresponding to the samples of basal urine and of urine taken during 3 hours from an individual with lactose tolerance and an individual with lactose intolerance are shown. The retention times of the peaks assigned to xylose and to the internal standard in each case are indicated in minutes. Fig. 7 shows that the xylose excreted in urine during 3 hours after the intake of 4-galactosyl-xylose by the hypolactasic individual 12 was likewise much lower than that excreted by the individual 1 with lactose tolerance, as one can see by the area of the peaks corresponding to xylose in the chromatograms. This process determines a total amount of 2.1 mg of xylose in urine of 3 hours in the hypolactasic individual 12 with lactose intolerance, in contrast to 25.2 mg total xylose in urine of 3 hours in the individual 1 with lactose tolerance what represented a 92% reduction that is in accordance with the intense intestinal lactase deficiency that this hypolactasic individual suffers from, and it is similar to the degree of reduction of xylose titrated colorimetrically after the same period of time in this same individual as one can see in Fig. 6A. This experiment shows that the methodology that is presented permits non-invasive diagnosis of intestinal lactase deficiency in humans irrespective of the method used for measuring the evaluation of excreted xylose.
**Examples 17 to 19**: The evaluation of xylose in blood after the oral administration of 4-galactosyl-xylose permits non-invasive diagnosis of intestinal lactase deficiency. This has been shown by administering 3 g of 4-galactosyl-xylose to two volunteers with lactose tolerance [individual 1, Example 17 and individual 2, Example 18 (these individuals were also studied in Examples 3 to 6)] and to the hypolactasic individual 12 with lactose intolerance [Example 19 (this individual was also studied in examples 12 and 15]). Xylose was titrated colorimetrically with fluoroglucinol in blood plasma of blood samples obtained from these individuals prior to the intake of the disaccharide and after the intake thereof at approximately 1 hour intervals for 6 hours: in the hypolactasic individual a basal blood sample and another blood sample 2 hours after the intake of the 4-galactosyl-xylose disaccharide were taken. The intake of 3 g of 4-galactosyl-xylose did not result in any gastrointestinal discomfort or disorder in this individual 12 with lactose intolerance or in the two individuals with lactose tolerance. The results of these experiments are compiled in Fig. 8, wherein one can see: 1) That xylose is present in blood plasma after oral administration of 4-galactosyl-xylose to individuals 1 and 2 with lactose tolerance, as a result of the hydrolysis of this disaccharide by intestinal lactase; 2) That the presence of xylose in the blood plasma of these two individuals reached a maximum about 2 hours after the intake of 4-galactosyl-xylose, and then it decreased until it was undetectable after 6 hours, as a result of the xylose passing into the urine; 3) That in the individual 12 with lactose tolerance xylose was detected in blood plasma coming from the blood sample taken 2 hours after the intake of 4-galactosyl-xylose in an amount of 0.48 µg/100µl of plasma, that in contrast to the amount detected in a similar period of time in individual 1 of 1.72 µg/100 ml of plasma and in individual 2 of 1.95 µg/100 ml of plasma, represented a reduction of xylose in plasma in the hypolactasic individual with lactose intolerance of 72% and 75% respectively. In view of the amount of xylose detected in the individual with lactose intolerance 2 hours after the intake of 4-galactosyl-xylose, 0.48 µg/100 µl of plasma and the sensitivity of the titration (minimum detection limit of xylose with fluoroglucinol of 0.1 to 0.05 µ), the minimum volume of serum or plasma to reliably make the measurement is estimated to be 50 µl, which is equivalent to 0.2 ml of blood (2 to 3 drops). These experiments show that the evaluation of xylose in blood after the oral administration of 4-galactosyl-xylose permits *in vivo* evaluation of intestinal lactase activity and it is a valid test for non-invasive diagnosis of the deficiency of this enzyme in individuals.

## Claims

1. Use of 4-galactosyl-xylose for the evaluation of the presence of xylose in urine, usable for *in vivo* evaluation of intestinal lactase as a diagnostic test of the deficiency of this enzyme, that comprises oral administration of an aqueous 4-galactosyl-xylose solution to an individual who has fasted for at least 8 hours and the evaluation of the amount of xylose present in urine.

2. Use according to claim 1, **characterized in that** the amount of xylose present in urine is calculated by means of the difference found between the measurement made in an aliquot coming from the total volume of urine collected after the intake of 4-galactosyl-xylose and the measurement made in an aliquot coming from the volume of basal urine collected immediately before the individual intakes this compound.

3. Use, according to claim 1, **characterized in that** the individual is a human being.

4. Use of 4-galactosyl-xylose to evaluate the presence of xylose in blood, useable for *in vivo* evaluation of intestinal lactase as a diagnostic test of the deficiency of this enzyme, which comprises oral administration of an aqueous 4-galactosyl-xylose solution to an individual who has fasted for at least 8 hours and the evaluation of the amount of xylose present in blood.

5. Use, according to claim 4, **characterized in that** the amount of xylose present in blood after the intake of 4-galactosyl-xylose can be evaluated in blood plasma or blood serum.

6. Use, according to claim 4, **characterized in that** the amount of xylose present in blood is calculated from the difference found between the measurement made in an aliquot of a fluid coming from a blood sample extracted from an individual after the intake of 4-galactosyl-xylose and the measurement made in an aliquot coming from a sample of basal blood extracted immediately before the intake of this compound by the individual.

7. Use according to claim 4, **characterized in that** the individual is a human being.

8. Use according to claims 1 and 4, **characterized in that** its use in human individuals permits carriers of intestinal lactase deficiency from being distinguished from those individuals who are allergic to some component of milk, but who do not have the deficiency of this enzyme.
